**Europäisches Patentamt**

**European Patent Office**

**Office européen des brevets**

(19)

(11) Veröffentlichungsnummer: **0 325 113**
**A1**

(12)

# EUROPÄISCHE PATENTANMELDUNG

(21) Anmeldenummer: **89100118.2**

(22) Anmeldetag: **05.01.89**

(51) Int. Cl.4: **A61K 31/71 , A61K 9/14 , A61K 47/00**

---

Claims for the following Contracting States: ES + GR.

(30) Priorität: **18.01.88 DE 3801178**

(43) Veröffentlichungstag der Anmeldung:
**26.07.89 Patentblatt 89/30**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Pohler, Wolfgang, Dr.**
**Martin-Wohmann-Strasse 27**
**D-6238 Hofheim am Taunus(DE)**

---

(54) **Stabilisierte Trockenzubereitung zytostatisch wirksamer Anthracyclin-Antibiotika und Verfahren zu ihrer Herstellung.**

(57) Es werden stabilisierte Trockenzubereitung zytostatisch wirksamer Anthracyclin-Antibiotika enthaltend als Stabilisierungsmittel Lactose und als Antibiotikum ein Rhodomycinon-Hexasaccharid oder ein Rhodomycinon-Tetrasaccharid sowie Verfahren zu deren Herstellung beschrieben.

EP 0 325 113 A1

## Stabilisierte Trockenzubereitung zytostatisch wirksamer Anthracyclin-Antibiotika und Verfahren zu ihrer Herstellung

Anthracyclin-Zytostatika sind zytotoxisch wirksame Antibiotika der Anthracyclin-Reihe. Es handelt sich um glucosidische Verbindungen mit einem Anthracen- bzw. Naphtacen-Grundgerüst. Die bekanntesten, in die Therapie eingeführten Vertreter dieser Gruppe sind Doxorubicin, Daunorubicin und Mitomycin.

Bekannt ist, daß Anthracyclin-Zytostatika chemisch relativ instabil sind. Neben ihrer Photolabilität unterliegen sie in wässriger Lösung einer hydrolytischen Zersetzung. Die im Handel befindlichen Darreichungsformen sind daher Trockenpräparate, überwiegend in Form von Lyophilisaten (vgl. Thoma, K., Dt. Apotheker Ztg. 127, 197 (1987), nachfolgend auch als L1 zitiert). Es wurde weiterhin beschrieben, daß Lyophilisate aufgrund ihrer großen inneren Oberfläche (Schwammgerüst) im Gegensatz zu Trockenpulvern eine höhere Lösungsgeschwindigkeit besitzen, die bei der Bereitung von Injektions- und Infusionslösungen dringend erwünscht ist, da die Lösungen wegen der Instabilität der Wirkstoffe unmittelbar vor Verabreichung hergestellt werden (vgl. L1). Zur Verbesserung der Gerüstbildung von Lyophilisaten werden bei niedrig dosierten Zubereitungen gerüstbildende Hilfsstoffe, wie z.B. geeignete Saccharide oder Aminosäuren, zugesetzt (vgl. z.B. L1 und Sucker, H. et al., Pharmazeutische Technologie, G. Thieme Verlag, Stuttgart, 1978, Seite 621, nachfolgend auch als L2 zitiert).

Es wurde festgestellt, daß Lyophilisate von Anthracyclin-Zytostatika insbesondere Rhodomycinon-Hexasaccharide und Tetrasaccharide, wie z.B. Rodorubicin (INN) mit dem Standardhilfsstoff Mannitol als Gerüstbildner selbst bei Kühlschranklagerung keine befriedigend Stabilität besitzen. Versuche, durch Verwendung von Glycin als Gerüstbildner oder durch Änderung des pH-Wertes der Zubereitung eine Verbesserung zu erreichen, zeigten nicht den gewünschten Erfolg; es wurde eine in ähnlichem Maße auftretende Zersetzung nach Lagerung festgestellt.

Aufgrund dieser Beobachtungen mußte der Fachmann erwarten, daß auch bei Verwendung anderer Gerüstbildner der gleichen Klasse (Saccharide oder Aminosäuren) keine stabileren Zubereitungen erhalten werden.

Es wurde nun überraschend gefunden, daß Lyophilisate mit Lactose als Hilfsstoff selbst nach 12 Monaten Raumtemperaturlagerung keine Zersetzung zeigen.

Dieser Befund kommt für den Fachmann völlig unerwartet, weil Lactose als Aldose weniger inert ist als z.B. Mannitol und es daher zu unerwünschten Reaktionen mit dem Wirkstoff (z.B. Maillard-Reaktion, vgl. z.B. List, P.H., Arzneiformenlehre, Wiss. Verlagsges. Stuttgart, 1982, Seiten 79, 109 und 476, nachfolgend auch als L3 zitiert) kommen kann.

Hinzu kommt, daß Lactose relativ schlechte Trocknungseigenschaften besitzt (vgl. L2). Nach Vakuumtrocknung bei Raumtemperatur enthalten Lactose-Träger meist 3 - 5 % Restwasser. Ein solch hoher Wassergehalt oder eine Trocknung bei höheren Temperaturen führt aber erfahrungsgemäß zur Zersetzung empfindlicher Wirkstoffe. Gemäß L2 und L3 sollte daher Lactose weder als Gerüstbildner noch als Stabilisator geeignet sein.

Die Erfindung betrifft daher Trockenzubereitungen von zytostatisch wirksamen Anthracyclin-Antibiotika, dadurch gekennzeichnet, daß sie ein Rhodomycinon-Hexasacchararid oder ein Rhodmycinon-Tetrasaccharid oder deren physiologisch verträgliche Säureadditionsalze als Wirkstoff und Lactose als Stabilisator enthalten.

Die Erfindung betrifft ferner ein Verfahren zur Herstellung solcher Trockenzubereitungen, dadurch gekennzeichnet, daß man den Wirkstoff mit Lactose trocken mischt oder Wirkstoff und Lactose gemeinsam in eine wäßrige Lösung bringt und anschließend lyophilisiert oder die Lösung einer Sprühtrocknung unterwirft.

Das Verfahren der Sprühtrocknung ist auch unter aseptischen Bedingungen zur Herstellung steriler Produkte möglich (vgl. P.H. List und P.C. Schmidt: Technologie pflanzlicher Arzneizubereitungen S. 354, Wiss. Verlagsgesellschaft, Stuttgart, 1984). Bei der Herstellung der Trockenzubereitung durch trockenes Mischen von Wirkstoff und Lactose wird zweckmäßigerweise amorphe bzw. teilamorphe Lactose verwendet, wobei die amorphe bzw. teilamorphe Lactose durch Gefriertrocknung oder Sprühtrocknung von Lactoselösungen zugänglich ist.

Rhodomycinon-Hexasaccharide und Rhodoemycinon-Tetrasaccharide, die in den erfindungsgemäßen Zubereitungen als Wirkstoffe enthalten sind, sind Gegenstand der Europäischen Patentanmeldungen EP-A-0131181 (Hexasaccharid) EP-A-0131942 und EP-A-0207463 (Tetrasaccharid).

Insbesondere enthalten die Zubereitungen das Rhodomycinon-Tetrasaccharid Rodorubicin (vgl. EP-A-0131942) oder das Rhodomycinon-Hexasaccharid, das in EP-A-0131181 als Cytorhodin A bezeichnet wird.

Die Wirkstoffe liegen gegebenenfalls im Gemisch mit pH-regulierenden Verbindungen vor. Die Zubereitung muß nach Auflösen in Wasser einen neutralen bis schwach sauren pH, zweckmäßigerweise zwischen etwa pH 2,5 und pH 7, aufweisen. Ein solches Medium liegt vor, wenn für die Salzbildung des Wirkstoffs ein Überschuß an Säure verwendet wird. Es kann auch zweckmäßig sein, einen entsprechenden Puffer, wie z.B. Citratpuffer, zuzusetzen.

Die Zubereitungen enthalten einen Überschuß an Lactose. Es ist zweckmäßig, den Zusatz an Lactose (Gerüstbildner/Stabilisator) so zu wählen, daß nach Auflösen in Wasser eine blutisotonische Lösung entsteht.

Die Zubereitungen können nach dem Auflösen in Wasser oder in einem pharmazeutisch geeigneten Lösungsmittel, z.B. physiologische Kochsalzlösung oder Glucose-Lösung, parenteral appliziert werden.

Die Erfindung betrifft daher auch die Verwendung der Trockenzubereitungen zur Herstellung von Injektionslösungen, indem das Lyophilisat in einen üblichen für Injektionszwecke geeigneten Lösungsmittel aufgenommen wird.

Die nachstehenden Beispiele zeigen, daß bei Verwendung von Lactose als Hilfsstoff stabile Zubereitungen resultieren, während bei Verwendung anderer Träger erhebliche Zersetzungsraten beobachtet werden.


**Beispiel 1**

Bei der Herstellung der Trockenzubereitungen werden Wirkstoffe, Lactose und Hilfsstoffe in Wasser gelöst und anschließend in an sich bekannter Weise (vgl. H. Sucker et al. (Hrsg.): Pharmazeutische Technologie, 1. Aufl., S. 198/199 und 620-628, Georg Thieme Verlag, Stuttgart, 1978 oder K.E. Avis et al. (Eds.): Parenteral Dosage Froms, Vol. 1, pp 245-251, Marcel Dekker Inc., New York/Basel 1984) lyophilisiert.

Die Zusammensetzungen der Trockenzubereitungen pro Ampulle sind der nachfolgenden Tabelle zu entnehmen. Sie ergeben nach Auflösen in 10 ml Wasser blutisotonische Lösungen.

| | | | | |
|---|---|---|---|---|
| Rodorubicin | 10 mg | 10 mg | | |
| Lactose | 1000 mg | 1000 mg | | |
| Gluconsäure q.s. | pH 6,5 | pH 3,5 | | |
| Gehalt an unzersetztem Wirkstoff nach 12 Monaten Raumtemp.-Lagerung | 99 % | 100 % | | |
| Vergleichspräparate | | | | |
| a) Rodorubicin | 0,1 mg | 1 mg | 10 mg | 10 mg |
| Mannitol | 500,0 mg | 500 mg | 500 mg | 500 mg |
| Gluconsäure zur pH-Einst. auf | pH 6,5 | pH 6,5 | pH 6,5 | pH 3,5 |
| Gehalt an unzersetztem Wirkstoff nach Raumtemp.-Lagerung | | | | |
| 6 Monate | 55 % | 59 % | - | - |
| 12 Monate | - | - | 89 % | 84 % |
| b) Rodorubicin | 10 mg | 10 mg | | |
| Glycin | 200 mg | 200 mg | | |
| Salzsäure q.s. | pH 6,5 | pH 3,5 | | |
| Gehalt an unzersetztem Wirkstoff nach 12 Monaten Raumtem.-Lagerung | 66 % | 86 % | | |

Zur Herstellung der Trockenzubereitung kann die obige wäßrige Lösung von Wirkstoff(en), Lactose und Hilfsstoffen anstelle der Lyophilisation auch einer Sprühtrocknung (vgl. P.H. List: Arzneiformenlehre, 3. Aufl., S. 280ff, Wiss. Verlagsgesellschaft, Stuttgart, 1982 oder E. A. Kenneth et al.: Pharmaceutical Dosage Forms-Parenteral Medications, Vol. 1, p. 176-178, Marcel Dekker Inc., New York/Basel, 1984) unterzogen und das resultierende Pulver in Ampullen oder Injektionsflaschen abgefüllt werden.

**Beispiel 2**

Cytorhodin A-Hydrochlorid, entsprechend 5 g Cytorhodin A und 995 g Lactose, amorph bzw. teilamorph werden gemischt. Die Mischung wird zu je 1 g in Injektionsflaschen gefüllt, welche mit Gummistopfen verschlossen und mit Aluminiumkappen zugebördelt werden. Die so erhaltenen Injektionsflaschen enthalten 5 mg Cytorhodin A und ergeben nach dem Auflösen des Inhaltes in Wasser eine isotonische Injektionslösung.

**Ansprüche**

1. Stabilisierte Trockenzubereitung zytostatisch wirksamer Anthracyclin-Antibiotika, dadurch gekennzeichnet, daß sie als Stabilisierungsmittel Lactose und als Antibiotikum ein Rhodomycinon-Hexasaccharid oder ein Rhodomycinon-Tetrasaccharid enthält.

2. Stabilisierte Trockenzubereitung nach Anspruch 1, dadurch gekennzeichnet, daß mindestens eine der folgenden Bedingungen zutrifft:

a) das Anthracyclin-Antibiotikum Rodorubicin oder Cytorhodin ist,

b) die Trockenzubereitung ein Lyophilisat oder ein Sprühtrocknungsprodukt ist,

c) die Trockenzubereitung zusätzlich eine pH-regulierende Verbindung enthält.

3. Stabilisierte Trockenzubereitung nach Anspruch 1, dadurch gekennzeichnet, daß sie Rodorubicin oder Cytorhodin A enthält und ein Lyophilisat ist.

4. Stabilisierte Trockenzubereitung gemäß Anspruch 1 zur Herstellung einer Injektionslösung.

5. Verfahren zur Herstellung einer Trockenzubereitung nach Anspruch 1, dadurch gekennzeichnet, daß sie durch trockenes Mischen der Anthracyclin-Antibiotika mit Lactose oder durch Herstellung einer gemeinsamen Lösung mit nachfolgender Lyophilisierung bzw. Sprühtrocknung erhalten wird.

6. Verwendung einer Trockenzubereitung gemäß Anspruch 1 zur Herstellung von Injektionslösungen.


Patentansprüche für folgende Vertragsstaaten: ES, GR.

1. Verfahren zur Herstellung einer stabilisierten Trockenzubereitung zytostatisch wirksamer Anthracyclin-Antibiotika, dadurch gekennzeichnet, daß sie durch trockenes Mischen eines Anthracyclin-Antibiotikums ausgewählt aus der Gruppe bestehend aus Rhodomycinon-Hexasaccharid und Rhodomycinon-Tetrasaccharid mit Lactose als Stabilisierungsmittel oder durch Herstellung einer gemeinsamen Lösung aus einem Antibiotikum ausgewählt aus der Gruppe bestehend aus Rhodomycinon-Hexasaccharid und Rhodomycinon-Tetrasaccharid und Lactose als Stabilisierungsmittel mit nachfolgender Lyophilisierung bzw. Sprühtrocknung erhalten wird.

2. Verfahren gemäß Anspruch 1, dadurch gekennzeichnet, daß mindestens eine der folgenden Bedingungen eingehalten wird:

a) als Anthracyclin-Antibiotikum Rodorubicin oder Cytorhodin verwendet wird,

b) die Trockenzubereitung durch Lyophilisierung oder Sprühtrocknung erhalten wird,

c) zusätzlich eine pH-regulierende Verbindung zugesetzt wird.

3. Verfahren gemäß Anspruch 1, dadurch gekennzeichnet, daß Rodorubicin oder Cytorhodin A eingesetzt wird und die Herstellung aus einer gemeinsamen Lösung mit nachfolgender Lyophilisierung erfolgt.

4. Verwendung einer Trockenzubereitung gemäß Anspruch 1 zur Herstellung von Injektionslösungen.

**Europäisches Patentamt**

**EUROPÄISCHER RECHERCHENBERICHT**

Nummer der Anmeldung

EP 89 10 0118

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,Y | EP-A-0 131 942  (HOECHST AG)<br>* Ansprüche 1,8 *<br>--- | 1-6 | A 61 K  31/71<br>A 61 K   9/14<br>A 61 K  47/00 |
| Y | DICTIONNAIRE VIDAL 1986, Auflage 62, Seiten 20,21, O.V.P. Paris, FR<br>* Seiten 20,21: "Adriblastine" *<br>--- | 1-6 | |
| A | GB-A-2 165 751  (FARMITALIA C. ERBA S.p.A.)<br>* Seite 1, Zeilen 6-70,123-130; Seite 2, Zeilen 10-16; Beispiel 1 *<br>--- | 1-6 | |
| A | CHEMICAL ABSTRACTS, Band 104, Nr. 6, 10. Februar 1986, Seite 382, Zusammenfassung Nr. 39716d, Columbus, Ohio, US; & JP-A-60 92 212 (KYOWA HAKKO KOGYO CO., LTD) 23-05-1985<br>* Zusammenfassung *<br>--- | 1-6 | |
| D,A | DEUTSCHE APOTHEKER ZEITUNG, Band 127, Nr. 5, 29. Januar 1987, Seiten 197-199, Deutscher Apotheker Verlag, Stuttgart, DE; K. THOMA: "Zytostatika: Wie sicher sind sie in der Handhabung?"<br>* Seite 197, Tabelle 2; Seite 198, Abschnitte:<br>"Lyophilisation","Zytostatische Antibiotika" *<br>--- | 1-6 | RECHERCHIERTE SACHGEBIETE (Int. Cl.4)<br><br>A 61 K |
| A | GB-A-2 178 311  (FARMITALIA C. ERBA S.p.A.)<br>* Seite 1, Zeilen 12,13,32-46,63,64; Seite 2, Zeilen 6-21; Seite 2, Zeile 65 - Seite 3, Zeile 8 *<br>--- | 2 | |
| A | DE-A-2 017 373  (ORSYMONDE S.A.)<br>* Seiten 5,6 *<br>---                              -/- | 5 | |

Der vorliegende Recherchenbericht wurde für alle Patentansprüche erstellt

| Recherchenort | Abschlußdatum der Recherche | Prüfer |
|---|---|---|
| DEN HAAG | 24-04-1989 | MUELLNERS W. |

Europäisches
Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 169 700 (ELI LILLY AND CO.)<br>* Seite 2, Zeilen 1-11; Seite 3, Zeilen 1-20; Seite 4, Zeile 22 - Seite 5, Zeile 13 *<br>----- | 5 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 24-04-1989 | MUELLNERS W. |

EPO FORM 1503 03.82 (P0403)